(19) Europäisches Patentamt
European Patent Office
Office européen des brevets

(11) **EP 1 660 154 B1**

(12) **EUROPÄISCHE PATENTSCHRIFT**

(45) Veröffentlichungstag und Bekanntmachung des Hinweises auf die Patenterteilung:
**27.04.2011 Patentblatt 2011/17**

(21) Anmeldenummer: **04741351.3**

(22) Anmeldetag: **02.08.2004**

(51) Int Cl.:
***A61M 1/16*** (2006.01)   ***A61M 1/34*** (2006.01)

(86) Internationale Anmeldenummer:
**PCT/EP2004/008650**

(87) Internationale Veröffentlichungsnummer:
**WO 2005/021067 (10.03.2005 Gazette 2005/10)**

(54) **BLUTBEHANDLUNGSVORRICHTUNG**

BLOOD TREATMENT DEVICE

DISPOSITIF DE TRAITEMENT SANGUIN

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.08.2003 DE 10339342**

(43) Veröffentlichungstag der Anmeldung:
**31.05.2006 Patentblatt 2006/22**

(73) Patentinhaber: **Fresenius Medical Care Deutschland GmbH**
**61352 Bad Homburg (DE)**

(72) Erfinder: **KRÄMER, Matthias**
**61381 Friedrichsdorf (DE)**

(74) Vertreter: **Ziermann, Oliver**
**Fresenius Medical Care**
**AG & Co. KGaA**
**Global Patents & IP**
**Frankfurter Strasse 6 - 8**
**66606 St. Wendel (DE)**

(56) Entgegenhaltungen:
**WO-A-01/32237    WO-A-98/23311**
**US-A- 5 744 031    US-A1- 2001 037 968**

**Beschreibung**

Die Erfindung betrifft das Gebiet von Blutbehandlungsvorrichtungen mit einem durch eine semipermeable Membran in zwei Kammern geteilten Blutreinigungselement, deren erste Kammer Teil eines Dialysierflüssigkeitskreislaufs und deren zweite Kammer Teil eines extrakorporalen Blutkreislaufs ist.

Derartige Geräte werden als Hämodialysegeräte in der Nierenersatztherapie eingesetzt. Durch die Hämodialysebehandlung kann einem Dialysepatienten einerseits überschüssiges Wasser durch einen Druckgradienten an der semipermeablen Membran entzogen werden. Andererseits werden dem Patienten über einen Konzentrationsgradienten an der Membran Stoffe wie z.B. Harnstoff und Kreatinin entzogen, die ansonsten über die gesunde Niere ausgeschieden werden. Bei anderen Stoffen, die im Blut immer in einer gewissen Konzentration vorhanden sind - wie z.B. Elektrolyte - werden in der Dialysierflüssigkeit meist Konzentrationen eingesetzt, die im Blutbild denen nicht nierenkranker Patienten entsprechen.

Dialysepatienten nehmen üblicherweise zwischen den Behandlungen über die Nahrung beträchtliche Mengen von Kalium auf. Eine übermäßige Aufnahme, besonders aber eine Kumulation von Kalium über längere Zeit durch nicht ausreichenden Kaliumentzug während der Dialyse führt zur Hyperkälamie (erhöhte Kaliumkonzentration in der extrazellulären Flüssigkeit).

Die extrazelluläre Kaliumkonzentration hat einen Einfluss auf das elektrische Ruhe-Membranpotential von Zellen. Daher wirkt sich diese Konzentration auf den Grad der elektrischen Erregbarkeit von allen erregbaren Zellen aus (Muskeln, Herzmuskel, Nerven). Insbesondere die Auswirkung auf die kardiale Elektrophysiologie ist von Bedeutung. Abnormale Werte können zu bedrohlichen Arrhythmien führen. Die Kaliumkonzentration wird daher vom Körper in engen Grenzen geregelt. Unter den pathophysiologischen Bedingungen eines Ausfalls der Niere muss die Dialysetherapie so gestaltet werden, dass kritische Zustände vermieden werden.

Normale Konzentrationen von Kalium liegen im Bereich von 3,5-4,5 mmol/l. Oberhalb von 5,5 mmol/l spricht man von einer Hyperkalämie; Konsequenzen sind ventrikuläre Arrhythmien, Kammerflimmern, bis hin zum Herzstillstand. Liegt der Wert unterhalb von 2,5 mmol/l, spricht man von einer schweren Hypokalämie. Symptome sind hier Muskelschwäche, atriale und ventrikuläre Arrhythmien, Verwirrungszustände und Desorientierung. Die Übergangsbereiche milder Hyperkalämie (4,5-5,5 mmol/l) und milder Hypokalämie (2,5-3,5 mmol/l) sind häufig weitgehend symptomfrei.

Die Kinetik der Kaliumkonzentration während der Dialyse ist deutlich komplexer als beispielweise die von Harnstoff. Während Harnstoff intra- und extrazellulär in etwa gleichen Konzentrationen vorliegt, ist die gesamte Kaliummenge von ca. 3500 mmol (50 mmol/kg) sehr ungleichmäßig verteilt: Ca. 98% sind intrazellulär, nur 2% extrazellulär. Dieses Ungleichgewicht zwischen intra- und extrazellulärer Konzentration wird zudem beeinflusst von Änderungen in der Konzentration anderer extrazellulärer Moleküle, insbesondere von $H^+$-Ionen, Bicarbonat, Glucose, Insulin. Auch adrenerge Stimulation und der Aldosteronspiegel beeinflussen das Konzentrationsverhältnis. Außerdem wird ein starker Rebound beobachtet, d.h. nach Ende der Dialyse steigt die Kaliumkonzentration im Blut aufgrund des zeitlich verzögerten Transfers vom intrazellulären in den extrazellulären Raum wieder erheblich an.

Der Entzug von ca. 90% der seit der vorgehenden Behandlung aufgenommenen Kaliummenge durch die intermittierende Dialyse gestaltet sich nicht immer einfach. Es kann nur ein relativ kleiner Konzentrationsgradient ausgenutzt werden. Initiale Kaliumkonzentrationen bei Dialysepatienten liegen meist zwischen ca. 3,6 und 7 mmol/l, in einigen Fällen auch darüber. Die Kaliumkonzentration der Dialysierflüssigkeit liegt zwischen 0 und 4 mmol/l, wobei meist mittlere Konzentrationen um 2 mmol/l verwendet werden. Die ausnutzbaren Gradienten zwischen Blut und Dialysierflüssigkeit liegen daher typischerweise bei 2-4 mmol/l am Beginn der Dialyse, reduzieren sich jedoch während der Behandlung schnell. Dadurch ist der mögliche Gesamtentzug limitiert. Bei größerer Kaliumaufnahme besteht das Risiko, der er sogar zu gering ist. Dadurch ergibt sich für den Patienten das Risiko der Hyperkalämie, die insbesondere direkt vor den Dialysebehandlungen besonders ausgeprägt sein kann.

Die Verwendung von Dialysierflüssigkeit mit geringer Kaliumkonzentration oder gar ohne Kalium ist nicht ohne Risiko. Die Kaliumkonzentration kann während der Dialyse auf kritisch niedrige Werte abfallen, d.h. es kann eine schwere Hypokalämie ausgelöst werden (auch wenn die Gesamtmenge von Kalium im Körper noch ausreichend oder gar zu groß ist).

Eine zu hohe Rate der Kaliumentfernung könnte Arrhythmien induzieren (J.P. Knochel: Clinical Expression of Potassium Disturbances, in "The Regulation of Potassium Balance", 1. Auflage, herausgegeben von D.W. Seldin, Giebisch G., 1989, S. 207-240; E.G. Lowrie und N.L. Lew: Death Risk in Hemodialysis Patients: The Predictive Value of Commonly Measured Variables and an Evaluation of Death Rate Differences Between Facilities, Am.J.Kidney Dis. 15, S. 458-482 (1990)). Bei Erhöhung der Entzugsrate wird das Verhältnis von intra- zu extrazellulärer Kaliumkonzentration größer. Dieses führt dann zu einer Hyperpolarisation der Zellen und damit zu einer Abnahme der Erregbarkeit. Dadurch dürfte dann die Wahrscheinlichkeit von Arrhythmien zunehmen.

Die insbesondere in der frühen Phase der Dialyse hohe Zufuhr an Bikarbonat zum Patienten bewirkt eine Verschiebung des Kalium von dem extra- zum intrazellulären Raum, die - zusätzlich zum Entzug von Kalium über den Dialysator - zu einer Reduktion der extrazellulären Konzentration beiträgt und damit das Hypokalämie-Risiko erhöht.

Es ist vorgeschlagen worden, die Dialysebehandlung mit einer Modellsimulation zu begleiten, um Aussagen über die Entfernung bestimmter Blutinhaltstoffe treffen zu können, wobei die Berechnungen durch Blutproben ergänzt werden (S. Stiller, H. Mann und F. Raab, Microprocessor based universal dialysis calculator for individualization of artificial kidney dialysis, MEDINFO 80, herausgegeben von Lindberg/Kaihara, North-Holland Publishing Company, 1980, S. 534-538).

US 4,244,787 beschreibt eine Vorrichtung, bei der über einen Sensor in der Dialysierflüssigkeitsabführleitung die Konzentration eines Stoffes in der Blutzuführleitung bestimmt werden kann. Gleichzeitig ist es möglich, die insgesamt entfernte Menge des Stoffes zu ermitteln.

US 4,508,622 beschreibt ein Hämodialysegerät, bei dem durch einen Sensor in der Dialysierflüssigkeitsabführleitung und einen gleichartigen Sensor in der Dialysierflüssigkeitszuführleitung die Elektrolytbilanz während einer Dialysebehandlung festgestellt werden kann und eine Rückkopplung auf die Dialysebehandlung zu ermöglichen. Eine ähnliche Vorrichtung ist Gegenstand der EP 0 330 892 A2.

WO 98/23311 beschreibt eine Blutbehandlungsvorrichtung, deren Auswerteeinheit geeignet ist, anhand der Messwerte mindestens eines Sensors die Konzentration von Harnstoff im Blut in der Blutzuführleitung sowie die insgesamt während der Behandlung durch die Membran entzogene Menge von Harnstoff zu bestimmen, wobei in der Auswerteeinheit ein Wert für die insgesamt zu entziehende Menge von Harnstoff hinterlegt ist, und die Auswerteeinheit so ausgelegt ist, die Steuereinheit dahingehend anzuweisen, dass die Blutbehandlungsvorrichtung die Blutbehandlung unter Einhaltung des Werts durchführt. Der Wert für die insgesamt zu entziehende Menge von Harnstoff ist dabei durch die Festlegung der gewünschten Dialysedosis definiert.

Der Erfindung liegt die Aufgabe zugrunde, eine Blutbehandlungsvorrichtung mit einem durch eine semipermeable Membran in zwei Kammern geteilten Blutreinigungselement, deren erste Kammer Teil eines Dialysierflüssigkeitskreislaufs und deren zweite Kammer Teil eines extrakorporalen Blutkreislaufs ist, dahingehend weiterzubilden, dass unphysiologische Zustände des Patienten während der Blutbehandlung besser vermieden werden können.

Die Lösung dieser Aufgabe gelingt mit einer Blutbehandlungsvorrichtung mit den Merkmalen des Anspruchs 1. Vorteilhafte Ausführungsformen der Erfindung sind Gegenstand der Unteranspüche.

Erfindungsgemäß ist vorgesehen, dass die Auswerteeinheit der Blutbehandlungsvorrichtung anhand mindestens eines Sensors in einem des Blutkreislauf oder Dialysierflüssigkeitskreislauf zur Erfassung der Konzentration eines Stoffes, der die semipermeable Membran durchdringen kann, die Konzentration dieses Stoffes im Blut in der Blutzuführleitung, die momentane Transferrate dieses Stoffes durch die Membran sowie die insgesamt während der Behandlung entzogene Menge diese Stoffes bestimmen, die Konzentration mit einem ersten zulässigen Wertebereich, die Transferrate mit einem zweiten zulässigen Wertebereich und die entzogen Menge mit einem dritten Wertebereich vergleichen und die die Blutbehandlungsvorrichtung steuernde Steuereinheit dahingehend anweisen kann, dass die Blutbehandlungsvorrichtung die Blutbehandlung unter Einhaltung aller drei zulässiger Wertebereiche durchführt.

Eine derartige Vorrichtung ermöglicht durch ihre erfindungsgemäße Gestaltung, dass kritische Substanzen wie z.B. Kalium während der Blutbehandlung in physiologisch vertretbaren Grenzen und Zeiträumen kontrolliert werden können.

In vorteilhafter Ausführungsform ist der mindestens eine Sensor in der Dialysierflüssigkeitsabführleitung angeordnet. Falls die Konzentration des betrachteten Stoffes in der Dialysierflüssigkeitszuführleitung nicht bekannt ist, kann dort ein weiterer, gleichartiger Sensor vorgesehen sein.

In gleichem Maße ist es möglich, dass derartige Sensoren auch im Blutkreislauf vorgesehen sind. Aufgrund der mit einem Blutkontakt einhergehenden Probleme sind Sensoren im Dialysierflüssigkeitskreislauf jedoch im Allgemeinen vorzuziehen.

In einer Weiterbildung der Erfindung ist in der Auswerteeinheit ein Zielwert innerhalb des dritten Wertebereiches hinterlegt, wobei die Auswerteeinheit die Steuereinheit dahingehend anweist, dass der Zielwert spätestens am Ende der geplanten Behandlungszeit erreicht wird.

Die Erfindung kann gleichermaßen in einer Hämodialyse- oder einer Hämofiltrationsbehandlung zur Anwendung kommen. In letzterem Fall mündet die Dialysierflüssigkeitzuführleitung als Substitutionszuführleitung direkt in den Blutkreislauf und die erste Kammer des Blutbehandlungselementes ist nur mit der Dialysierflüssigkeitsabführleitung als Filtratabführleitung verbunden. Damit ist insbesondere auch die Anwendung bei dem kombinierten Behandlungsverfahren, der Hämodiafiltration, möglich. Im Folgenden wird der Einfachheit halber jedoch nur von den Komponenten des eines Dialysierflüssigkeitskreislauf gesprochen, ohne dass dies auf die Hämodialyse einschränkend zu interpretieren ist.

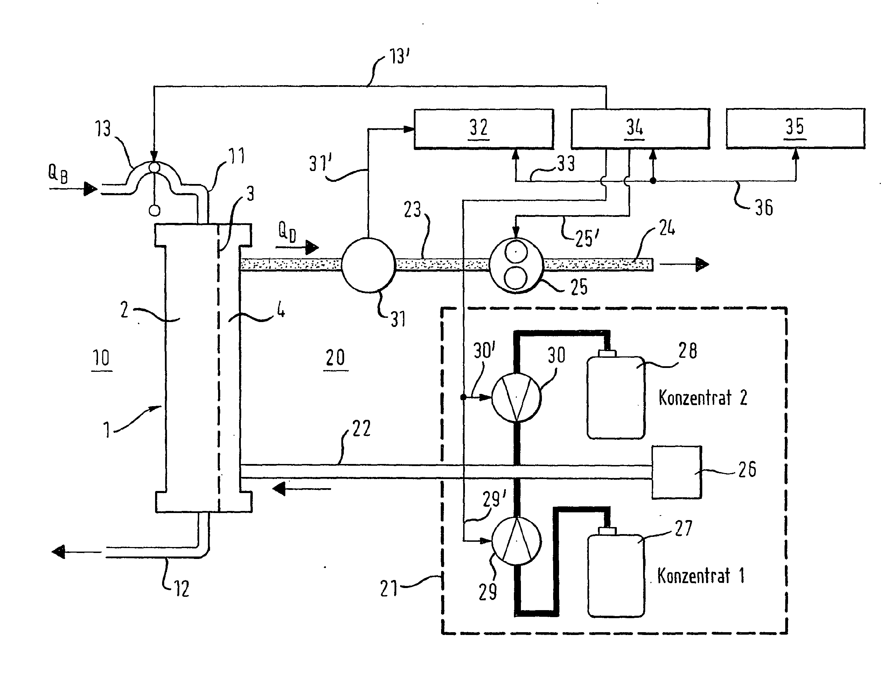

Weitere Einzelheiten und Vorteile der Erfindung werden anhand eines in der Zeichnung schematisch dargestellten Ausführungsbeispiels näher beschrieben.

Die in der einzigen Zeichnung dargestellte Blutbehandlungsvorrichtung weist einen extrakorporalen Blutkreislauf 10 und einen Dialysierflüssigkeitskreislauf 20 auf. In dem Blutkreislauf 10 wird Blut von einem Patienten (nicht gezeigt) über eine Blutzuführleitung 11 zu einer zweiten Kammer 2 eines durch eine semipermeable Membran 3 in zwei Kammern 2 und 4 geteilten Blutbehandlungselements 1 geleitet. Von der zweiten Kammer 2 gelangt das Blut über die Blutrückführleitung 12 wieder zu dem Patienten zurück. Das Blut wird in dem extrakorporalen Blutkreislauf 10 durch eine Blutpumpe 13 zirkuliert.

In dem Dialysierflüssigkeitskreislauf 20 gelangt Dialysierflüssigkeit von einer Dialysierflüssigkeitaufbereitungseinheit 21 über die Dialysierflüssigkeitszuführleitung 22 zur ersten Kammer 4 des Blutbehandlungselements 1. Die Dialysierflüssigkeit wird aus der ersten Kammer 4 über die Dialysierflüssigkeitsabführleitung 23 zu einem Abfluss 24 geleitet. Hierbei wird die Dialysierflüssigkeit mit der Dialysierflüssigkeitspumpe 25 zirkuliert.

Die Dialysierflüssigkeitsaufbereitungseinheit 21 stellt aus Wasser oder aus mit bereits einigen Komponenten der Dialysierflüssigkeit vermischtem Wasser (Anschluss 26) sowie einem Kaliumkonzentrat 27 oder zwei Kaliumkonzentraten 27 und 28 über die Konzentratpumpen 29 und 30 die zu verwendende Dialysierflüssigkeit nach vorgegebenen Mischungsverhältnissen her. Dabei kann auf bewährte und dem Fachmann geläufige Komponenten zurückgegriffen werden, die hier nicht im Einzelnen erörtert zu werden brauchen. Im Rahmen der Erfindung ist es nur wesentlich, dass die Beimengung des Kaliumkonzentrates in der Dialysierflüssigkeitsaufbereitungseinheit 21 nach der Vorgabe von Sollwerten eingestellt werden kann.

Eine besonders vorteilhafte Realisierung zur Bereitstellung von Dialysierflüssigkeit mit einer unteren und einer oberen Begrenzung der Kaliumkonzentration besteht darin, mit zwei Konzentraten 27 und 28 zu arbeiten, wobei das erste Konzentrat 27 eine niedrige Kaliumkonzentration und das zweite Konzentrat 28 eine höhere Kaliumkonzentration aufweist. Sollten diese Konzentrate auch andere Stoffe wie z.B. die saure Komponente der Dialysierflüssigkeit und andere Elektrolyte enthalten, so sind diese zweckmäßigerweise in beiden Konzentraten in gleicher Konzentration enthalten. Dann kann bei einer konstanten Beimengungsrate, die die Summe des durch die Konzentratpumpen 29 und 30 geförderten Konzentratflusses ist, die Kaliumkonzentration nie einen bestimmten Konzentrationsbereich in der Dialysierflüssigkeit verlassen.

In der Dialysierflüssigkeitsabführleitung 23 ist ein Kalium empfindlicher Sensor 31 vorgesehen, der über eine Leitung 31' mit einer Auswerteeinheit 32 verbunden ist. Die Auswerteeinheit 32 ist ihrerseits über eine Datenleitung 33 mit einer Steuereinheit 34 verbunden. Die Auswerte- und Steuereinheit 32 und 34 können dabei als eine einzige, integrierte Einheit aufgebaut sein. Die Steuereinheit 34 ist mit den Aktoren und Sensoren der Blutbehandlungsvorrichtung verbunden, um die Blutbehandlung als solche gesteuert durchführen zu können. Zur Erläuterung der Erfindung sind dies im Besonderen die Konzentratpumpen 29 und 30 sowie die Dialysierflüssigkeitspumpe 25 und die Blutpumpe 13, die durch entsprechende Steuerleitungen 13', 25', 29' und 30' mit der Steuerleitung verbunden sind.

Für die Gestaltung weiterer in einer solchen Blutbehandlungsvorrichtung üblicher Komponenten wie Temperier-, Überwachungs- und Bilanzeinheiten sind dem Fachmann mannigfaltige Ausgestaltungen bekannt, auf die an dieser Stelle nicht näher eingegangen wird.

In der Auswerteeinheit 32 sind nun erfindungsgemäß drei zulässige Wertebereiche für die Konzentration Cbi von Kalium in der Blutzuführleitung 11, für die Transferrate $\Delta M/\Delta t$ von Kalium durch die semipermeable Membran 3 sowie die insgesamt entzogene Menge M hinterlegt.

Für den ersten Bereich liegt die minimale Begrenzung vorzugsweise bei 2,5-3,5 mmol/l, die maximale Begrenzung bei 4,5-5,5 mmol/l. Es kann auch vorgesehen sein, einen engeren ersten Wertebereich und einen diesen umfassenden weiteren ersten Wertebereich vorzusehen, damit in Form von zwei Alarmstufen einer milderen und einer schwereren Form von Hyper- und Hypokaliämie begegnet werden kann.

Für den zweiten zulässigen Wertebereich wird zweckmäßigerweise eine untere Grenze von 0 mmol/min festgesetzt, da dem Patienten im Allgemeinen Kalium zu entziehen ist. Es ist aber auch möglich, eine negative untere Grenze zu wählen, falls der Verdacht besteht, dass der Patient bereits am Beginn der Behandlung hypokalämisch ist.

Bei dem dritten zulässigen Wertebereich ist in der Auswerteeinheit 32 zunächst ein Zielwert Mend für die insgesamt zu entziehende Kaliummenge hinterlegt. Diese kann von einer Bedienperson über eine nicht näher gezeigte Schnittstelle eingegeben oder von der Auswerteeinheit anhand von Vergleichsdaten selbst festgelegt worden sein. Der dritte zulässige Wertebereich wird im einfachsten Fall einfach durch eine untere Grenze von 0 mol und durch die obere Grenze Mend - also gleich dem Zielwert - festgelegt.

Während der Blutbehandlung laufen die folgenden Prozesse in der Blutbehandlungsvorrichtung ab:

Die Dialysebehandlung wird mit einer üblichen Zusammensetzung der Dialysierflüssigkeit und üblichen Werten für den Blutfluss Qb und den Dialysierflüssigkeitsfluss Qd begonnen. Die Auswerteeinheit 32 entnimmt nach einer Messphase von wenigen Minuten, nachdem sich stabile Messwerte eingestellt haben, dem Sensor 31 den Konzentrationswert Cdo von Kalium in der Dialysierflüssigkeitsabführleitung 25. Des Weiteren ist der Auswerteeinheit 32 die Konzentration Cdi von Kalium in der Dialysierflüssigkeitszuführleitung 22 durch die Vorgabewerte der Steuereinheit 34 bekannt. Hierfür kann auch in der Dialysierflüssigkeitszuführleitung 22 ein zweiter, gleichartiger Sensor vorgesehen sein. Die Auswerteeinheit 32 bestimmt mit diesen Angaben die Blutkonzentration Cbi von Kalium in der Blutzuführleitung 11 mit Hilfe der folgenden Beziehung (J.A. Sargent und F.A. Gotch: Principles and biophysics, in: Replacment of Renal Function by Dialysis, herausgegeben von C. Jacobs et al., Kluwer Academic Publishers, Dordrecht, 1996, S. 34ff):

$$Cbi = \frac{Qd(Cdo - Cdi)}{\alpha D} + \frac{Cdi}{\alpha} \qquad (1),$$

wobei D die Dialysance und $\alpha$ der Gibbs-Donnan Koeffizient für Kalium ist. Der Gibbs-Donnan Koeffizient berücksichtigt den Ionen-Charakter von Kalium für die Transporteigenschaften durch eine semipermeable Membran, wobei in erster Näherung $\alpha=1$ gesetzt werden kann. Andernfalls wird ein entsprechend anderer ermittelter Wert in der Auswerteeinheit 32 hinterlegt.

Die Dialysance D kann vom Benutzer vorher in die Auswerteeinheit eingegeben worden sein, oder sie wurde initial mit den dazu bekannten Methoden von der Blutbehandlungsvorrichtung gemessen. Hierzu kann z.B. die in der US 5,100,554 beschriebene Technik verwendet werden. Dabei braucht nicht direkt die Dialysance für Kalium bestimmt werden. Es ist ausreichend, die Dialysance für einen anderen Stoff zu bestimmen, die mit der Dialysance von Kalium in einer festen Beziehung steht, wie dies Gegenstand der deutschen Patentanmeldung 10317024.3 ist.

Für die Bestimmung von Cbi können auch andere wie z.B. das in US 6,126,831 erläuterte Verfahren verwendet werden.

In Gleichung (1) ist der Einfachheit halber der Ultrafiltratfluss Qf, also der Nettofluss durch die semipermeable Membran 3 von der zweiten Kammer 2 zur ersten Kammer 4, gleich Null gesetzt worden. Dem Fachmann sind dabei modifizierte Gleichungen geläufig, die eine nicht verschwindende Ultrafiltrationsrate Qf berücksichtigen.

Nach der kurzen initialen Messphase vergleicht die Auswerteeinheit 32 den initialen Messwert von Cbi mit dem hinterlegten ersten zulässigen Wertebereich. Sollte der Wert bereits außerhalb des zulässigen Wertebereichs liegen, gibt die Auswerteeinheit 32 über die Steuereinheit 34 ein Alarmsignal ab und macht über eine Anzeige und Eingabeeinheit 35, die mit der Steuereinheit über eine Datenleitung 36 verbunden ist, auf die kritische Blutkonzentration aufmerksam. Die Blutbehandlung wird automatisch gestoppt, so dass das Bedienpersonal die weiteren Schritte veranlassen kann.

Liegt der Messwert innerhalb des ersten zulässigen Bereichs, legt die Auswerteeinheit anhand der Grenzen des ersten und zweiten zulässigen Bereichs und ggf. des dritten zulässigen Bereichs die weiteren Behandlungsparameter fest, damit der Zielwert Mend der insgesamt zu entziehenden Kaliummenge innerhalb des dritten zulässigen Wertebereichs möglichst schnell ohne Verlassen aller zulässigen Wertebereiche erreicht werden kann. Dazu wird die Dialysierflüssigkeitskonzentration auf einen Wert eingestellt, der nahe an der unteren Grenze des ersten Wertebereichs liegt oder zumindest von dem momentanen Konzentrationswert durch eine Mindestdifferenz beabstandet ist. Gegebenenfalls kann auch der Dialysierflüssigkeitsfluss Qd oder der Blutfluss Qb erhöht werden. Hierdurch erhöht sich die Dialysance und damit die Transferrate $\Delta M/\Delta t$. Hierbei kann die Auswerteeinheit entweder empirisch vorgehen oder anhand bekannter Beziehungen der Dialysance von den Konzentrationen und den Flüssen - wie z.B. in der DE 10212247.4 diskutiert - die benötigte Zeit für den Entzug der Menge Mend abschätzen, so dass Behandlungsparameter vorgeschlagen werden können, die einen vollständigen Entzug innerhalb der gesamten Behandlungszeit T ermöglichen.

Nach dieser initialen Phase beginnt die eigentliche Blutbehandlung. In regelmäßigen Zeitabständen übermittelt der Sensor 31 Messwerte an die Auswerteeinheit 32. Dieser bestimmt die Blutkonzentration Cbi(t) wie bereits beschrieben sowie nun auch die Transferrate $\Delta M(t)/\Delta t$ und die insgesamt entzogene Kaliummenge M(t) mit Hilfe der Gleichungen (2) und (3):

$$\frac{\Delta M(t)}{\Delta t} = Qd(t) \cdot (Cdo(t) - Cdi(t)) \qquad (2),$$

$$M(t) = \int_0^t Qd(t') \cdot (Cdo(t') - Cdi(t')) \cdot dt' \qquad (3).$$

Die Auswerteeinheit 32 vergleicht alle drei Werte mit den jeweils zulässigen Wertebereichen. Besteht die Gefahr, dass der erste oder zweite Bereich verlassen wird, gibt die Auswerteeinheit 32 der Steuereinheit 34 entsprechende Anweisungen, so dass dem Verlassen der Wertebereiche entgegengewirkt werden kann. Sinkt z.B. der Wert für Cbi(t) zu sehr, so kann die Konzentration von Cdi(t) entsprechend gehoben werden. Es ist auch möglich, den Blutfluss Qb(t) oder den Dialysierflüssigkeitsfluss Qd(t) zu erniedrigen. Gleiches gilt, wenn die Transferrate $\Delta M(t)/\Delta t$ zu hoch wird.

Bei Erreichen des Zielwertes Mend=M(t), spätestens aber bei Erreichen der oberen Grenze des dritten Wertebereiches - falls diese Werte aus irgendeinem Grund voneinander abweichen sollten - wird die Steuereinheit 34 dahingehend angewiesen, die Kaliumkonzentration Cdi(t) auf den dem Blutwert Cbi(t) entsprechenden Wert einzustellen, so dass es bis zum Ende der Blutbehandlung zu keinem weiteren Entzug von Kalium mehr kommt.

Sollte die Behandlungzeit T abgelaufen sein, ohne dass die Zielmenge Mend trotz maximaler Transferrate erreicht werden konnte, so wird dies dem Benutzer entsprechend angezeigt. Hierzu können dem Benutzer bereits bei der Eingabe der Behandlungsdaten Informationen angezeigt werden, da sich die maximal während der Behandlungszeit T zu entziehende Menge eines Stoffes durch die obere Grenze für die Transferrate multipliziert mit der Behandlungszeit T ergibt.

Alle zwischenzeitlich berechneten Werte werden dem Bedienpersonal über die Anzeige- und Eingabeeinheit 35 zur Kenntnis gebracht. Dabei können unterschiedlichste graphische Hilfsmittel eingesetzt werden, um die einzelnen Werte in Relation zu den zulässigen Wertebereichen und einem sich eventuell anbahnenden Verlassen der Wertebereiche übersichtlich anzuzeigen. Besonders vorteilhaft ist dabei eine aktualisierte Anzeige direkt nach der initialen Bestimmung der Kaliumkonzentration, weil durch diesen Wert bereits Aussagen über die Verträglichkeit der Behandlung und eine eventuelle individualisierte Justierung der einzelnen Wertebereiche ermöglicht wird. Dabei kann die Auswerteeinheit 32 aufgrund von hinterlegten Beziehungen auch mit anderen patientenspezifischen Daten wie z.B. Körpergröße und Gewicht Vorschläge über die einzelnen Wertebereiche unterbreiten.

Durch die erfindungsgemäße Gestaltung der Blutbehandlungseinheit wird vermieden, dass die Blutkonzentration und die Transferraten eines die semipermeable Membran eines Blutbehandlungsgerätes durchdringenden Stoffes vorgegebene physiologische Bereiche verlassen kann, wobei gleichzeitig eine Therapie im Sinne einer zu entziehenden Menge dieses Stoffes unter priorisierter Einhaltung der Grenzen für Blutkonzentration und Transferrate ermöglicht wird.

## Patentansprüche

1. Blutbehandlungsvorrichtung mit einem durch eine semipermeable Membran (3) in zwei Kammern geteilten Blutreinigungselement (1), deren erste Kammer (4) Teil eines Dialysierflüssigkeitskreislaufs (20) und deren zweite Kammer (2) Teil eines extrakorporalen Blutkreislaufs (10) ist,

   mit einer von einer Dialysierflüssigkeitsaufbereitungseinheit (21) führenden Dialysierflüssigkeitszuführleitung (22) zur Zuführung frischer Dialysierflüssigkeit zur ersten Kammer (4) und/oder direkt in den Blutkreislauf (10),

   mit einer Dialysierflüssigkeitsabführleitung (23) zur Abführung verbrauchter Dialysierflüssigkeit aus der ersten Kammer (4),

   mit einer Blutzuführleitung (11) zur Zuführung von Blut zur zweiten Kammer (2),

   mit einer Blutrückführleitung (12) zur Rückführung von Blut aus der zweiten Kammer (2),

   mit einer Steuereinheit (34) zur Steuerung der Blutbehandlungvorrichtung,

   mit einer mit der Steuereinheit (34) verbundenen Auswerteeinheit (32),

   mit mindestens einem mit der Auswerteeinheit (32) verbundenen Sensor (31) an mindestens einem des Blutkreislauf (10) oder Dialysierflüssigkeitskreislauf (20) zur Erfassung der Konzentration eines Stoffes, der die semipermeable Membran (3) durchdringen kann,

   wobei die Auswerteeinheit (32) geeignet ist, anhand der Messwerte des mindestens einen Sensors (31) die Konzentration Cbi dieses Stoffes im Blut in der Blutzuführleitung (11), die momentane Transferrate $\Delta M/\Delta t$ dieses Stoffes durch die Membran (3) sowie die insgesamt während der Behandlung durch die Membran (3) entzogene Menge M diese Stoffes zu bestimmen,

   wobei in der Auswerteeinheit (32) ein erster zulässiger Wertebereich für die Blutkonzentration Cbi des Stoffes, ein zweiter zulässiger Wertebereich für die Transferrate $\Delta M/\Delta t$ sowie ein dritter zulässiger Wertebereich für die insgesamt zu entziehende Menge M des Stoffes hinterlegt sind, und

   wobei die Auswerteeinheit (32) so ausgelegt ist, die Steuereinheit (34) dahingehend anzuweisen, dass die Blutbehandlungsvorrichtung die Blutbehandlung unter Einhaltung aller drei zulässiger Wertebereiche durchführt.

2. Blutbehandlungsvorrichtung nach Anspruch 1, **dadurch gekennzeichnet, dass** der mindestens eine Sensor (31) in der Dialysierflüssigkeitsabführleitung (23) zur Bestimmung der Konzentration Cdo angeordnet ist.

3. Blutbehandlungsvorrichtung nach Anspruch 2, **dadurch gekennzeichnet, dass** ein zweiter Sensor in der Dialysierflüssigkeitszuführleitung (22) zur Bestimmung der Konzentration Cdi des Stoffes angeordnet ist, der ebenfalls mit der Auswerteeinheit (32) verbunden ist.

4. Blutbehandlungsvorrichtung nach Anspruch 2, **dadurch gekennzeichnet, dass** die Steuer- (34) und/oder die Auswerteeinheit (32) derart ausgelegt ist, dass sie die Konzentration Cdi des Stoffes in der Dialysierflüssigkeitszuführleitung (22) vorgibt.

5. Blutbehandlungsvorrichtung nach einem der vorhergehenden Ansprüche, **dadurch gekennzeichnet, dass** der Stoff Kalium ist.

6. Blutbehandlungsvorrichtung nach einem der vorhergehenden Ansprüche, **dadurch gekennzeichnet, dass** sich der zweite Wertebereich von null bis zu einem maximalen Wert erstreckt.

7. Blutbehandlungsvorrichtung nach einem der vorhergehenden Ansprüche, **dadurch gekennzeichnet, dass** in der Auswerteeinheit (32) ein innerhalb des dritten Wertebereichs liegender Zielwert Mend für die insgesamt zu entziehende Menge hinterlegt ist.

8. Blutbehandlungsvorrichtung nach Anspruch 7, **dadurch gekennzeichnet, dass** die Auswerteeinheit (32) derart ausgelegt ist, dass sie die Steuereinheit (34) dahingehend anweist, dass der Zielwert Mend nach einer geplanten Behandlungszeit erreicht wird.

9. Blutbehandlungsvorrichtung nach Anspruch 7, **dadurch gekennzeichnet, dass** die Auswerteeinheit (32) derart ausgelegt ist, dass sie die Steuereinheit (34) dahingehend anweist, bei Erreichen des Zielwertes Mend die Blutbehandlung mit einer Konzentration Cdi des Stoffes in der Dialysierflüssigkeitszuführleitung (22) fortzuführen, so dass es zu keinem Transfer des Stoffes durch die Membran (3) mehr kommt.

10. Blutbehandlungsvorrichtung nach einem der vorhergehenden Ansprüche, **dadurch gekennzeichnet, dass** die Steuereinheit (34) geeignet ist, mit voreingestellten Behandlungsparametern eine initiale Messung der Blutkonzentration Cbi zu veranlassen und die Auswertewerteeinheit (32) geeignet ist, den initialen Wert von Cbi zu bestimmen und unter Berücksichtigung dieses Wertes, des ersten zulässigen Wertebereichs und des zweiten zulässigen Wertebereichs für die Blutbehandlung einen Wert für die Konzentration Cdi des Stoffes in der Dialysierflüssigkeitszuführleitung (22), des Dialysierflüssigkeitsfluss Qd und/oder des Blutfluss Qb vorzuschlagen.

11. Blutbehandlungsvorrichtung nach Anspruch 10, **dadurch gekennzeichnet, dass** die Auswerteeinheit (32) derart ausgelegt ist, dass sie die Konzentration Cdi durch den der unteren Grenze des ersten zulässigen Wertebereiches entsprechenden Wert bestimmt.

12. Blutbehandlungsvorrichtung nach Anspruch 10, **dadurch gekennzeichnet, dass** die Auswerteeinheit (32) derart ausgelegt ist, dass sie die Konzentration Cdi durch die obere Grenze des zweiten zulässigen Wertebereiches bestimmt.

13. Blutbehandlungsvorrichtung nach Anspruch 11 und 12, **dadurch gekennzeichnet, dass** an einer Eingabeeinrichtung (35) Auswahlmittel über eine Priorisierung der Entziehung durch eine Ausrichtung an der unteren Grenze des ersten zulässigen Wertebereichs oder der oberen Grenze des zweiten zulässigen Wertebereichs vorgesehen sind.

**Claims**

1. A blood treatment device having a blood purification element (1), which is divided into two chambers by a semipermeable membrane (3), its first chamber (4) being part of a dialysis fluid circuit (20) and its second chamber (2) being part of an extracorporeal blood circuit (10),
having a dialysis fluid inlet line (22), which leads from a dialysis fluid processing unit (21) to supply fresh dialysis fluid to the first chamber (4) and/or directly into the blood circuit (10),
having a dialysis fluid outlet line (23) for removing spent dialysis fluid from the first chamber (4),
having a blood inlet line (11) for supplying blood to the second chamber (2),
having a blood return line (12) for returning blood from the second chamber (2),
having a control unit (34) for controlling the blood treatment device,
having an analyzer unit (32), which is connected to the control unit (34),
having at least one sensor (31), which is connected to the analyzer unit (32) on at least one blood circuit (10) or dialysis fluid circuit (20) for detecting the concentration of a substance, which is capable of penetrating through the semipermeable membrane (3),
whereby the analyzer unit (32) is suitable for determining on the basis of the measured values of the at least one sensor (31) the concentration Cbi of this substance in the blood in the blood inlet line (11), the instantaneous transfer

rate $\Delta M/\Delta t$ of this substance through the membrane (3) and the total quantity M of this substance withdrawn through the membrane (3) during the treatment,

whereby a first admissible value range for the blood concentration Cbi of the substance, a second admissible value range for the transfer rate $\Delta M/\Delta t$ and a third admissible value range for the total quantity M of the substance to be withdrawn are stored in the analyzer unit (32), and

whereby the analyzer unit (32) is designed so that it instructs the control unit (34) to the extent that the blood treatment device performs the blood treatment while maintaining all three admissible value ranges.

2. The blood treatment device according to Claim 1, **characterized in that** the at least one sensor (31) is provided in the dialysis fluid outlet line (23) for determining the concentration Cdo.

3. The blood treatment device according to Claim 2, **characterized in that** a second sensor is provided in the dialysis fluid inlet line (22) for determining the concentration Cdi of the substance and is also connected to the analyzer unit (32).

4. The blood treatment device according to one of the preceding claims, **characterized in that** the control unit (34) and/or the analyzer unit (32) is/are designed so that they predetermine the concentration Cdi of the substance in the dialysis fluid inlet line (22).

5. The blood treatment device according to one of the preceding claims, **characterized in that** the substance is potassium.

6. The blood treatment device according to one of the preceding claims, **characterized in that** the second value range extends from zero up to a maximum value.

7. The blood treatment device according to one of the preceding claims, **characterized in that** a target value Mend within the third value range for the total quantity of the substance to be withdrawn is stored in the analyzer unit (32).

8. The blood treatment device according to Claim 7, **characterized in that** the analyzer unit (32) is designed so that it instructs the control unit (34) that the target value Mend has been reached after a planned treatment time.

9. The blood treatment device according to Claim 7, **characterized in that** the analyzer unit (32) is designed so that it instructs the control unit (34) that on reaching the target value Mend, the blood treatment is to be continued with a concentration Cdi of the substance in the dialysis fluid inlet line (22) such that there is no longer any transfer of the substance through the membrane (3).

10. The blood treatment device according to one of the preceding claims, **characterized in that** the control unit (34) is suitable for ordering an initial measurement of the blood concentration Cbi with preset treatment parameters, and the analyzer unit (32) is suitable for determining the initial value of Cbi, and taking into account this value, the first admissible value range and the second admissible value range for the blood treatment, proposing a value for the concentration Cdi of the substance in the dialysis fluid inlet line (22), the dialysis fluid flow Qd and/or the blood flow Qb.

11. The blood treatment device according to Claim 10, **characterized in that** the analyzer unit (32) is designed so that it determines the concentration Cdi based on the value corresponding to the lower limit of the first admissible value range.

12. The blood treatment device according to Claim 10, **characterized in that** the analyzer unit (32) is designed so that it determines the concentration Cdi based on the upper limit of the second admissible value range.

13. The blood treatment device according to Claims 11 and 12, **characterized in that** selection means regarding a prioritization of the withdrawal (of the substance) are provided on the input device (35) by an alignment with the lower limit of the first admissible value range or the upper limit of the second admissible value range.

**Revendications**

1. Dispositif de traitement du sang comprenant un élément de purification du sang (1) divisé en deux chambres par une membrane semiperméable (3), dont la première chambre (4) fait partie d'un circuit de liquide de dialyse (20) et dont la deuxième chambre (2) fait partie d'un circuit sanguin (10) extracorporel,

comprenant une tubulure d'alimentation de liquide de dialyse (22) partant d'une unité de préparation du liquide de dialyse (21) pour apporter du liquide de dialyse neuf à la première chambre (4) et/ou directement dans le circuit sanguin (10),

comprenant une tubulure d'évacuation de liquide de dialyse (23) pour évacuer le liquide de dialyse usagé de la première chambre (4),

comprenant une tubulure d'alimentation du sang (11) pour amener du sang vers la deuxième chambre (2),

comprenant une tubulure de retour du sang (12) pour renvoyer le sang de la deuxième chambre (2),

comprenant une unité de commande (34) pour commander le dispositif de traitement du sang,

comprenant une unité d'évaluation (32) reliée à l'unité de commande (34),

comprenant au moins un capteur (31) relié à l'unité d'évaluation (32) sur au moins l'un du circuit sanguin (10) ou du circuit de liquide de dialyse (20) pour détecter la concentration d'une substance qui peut pénétrer la membrane semiperméable (3),

sachant que l'unité d'évaluation (32) est adaptée pour déterminer, à l'aide des valeurs de mesure de l'au moins un capteur (31), la concentration Cbi de cette substance dans le sang dans la tubulure d'alimentation du sang (11), les taux de transfert momentanés $\Delta M/\Delta t$ de cette substance à travers la membrane (3) ainsi que la quantité M totale de cette substance retirée de la membrane (3) pendant le traitement,

sachant que figurent dans l'unité d'évaluation (32), une première plage de valeurs autorisée pour la concentration sanguine Cbi de la substance, une deuxième plage de valeurs autorisée pour les taux de transfert $\Delta M/\Delta t$ ainsi qu'une troisième plage de valeurs autorisée pour la quantité M totale à retirer de la substance,

sachant que l'unité d'évaluation (32) est ainsi conçue pour ordonner à l'unité de commande (34) que le dispositif de traitement du sang conduit le traitement du sang en respectant les trois plages de valeurs autorisées.

2. Dispositif de traitement du sang selon la revendication 1, **caractérisé en ce que** l'au moins un capteur (31) est placé dans la tubulure d'évacuation de liquide de dialyse (23) pour déterminer la concentration Cdo.

3. Dispositif de traitement du sang selon la revendication 2, **caractérisé en ce qu'**un deuxième capteur est placé dans la tubulure d'alimentation de liquide de dialyse (22) pour déterminer la concentration Cdi de la substance et est relié le cas échéant à l'unité d'évaluation (32).

4. Dispositif de traitement du sang selon la revendication 2, **caractérisé en ce que** l'unité de commande (34) et/ou d'évaluation (32) est/sont ainsi conçue(s) qu'elle(s) prédétermine(nt) la concentration Cdi de la substance dans la tubulure d'alimentation de liquide de dialyse (22).

5. Dispositif de traitement du sang selon l'une des revendications précédentes, **caractérisé en ce que** la substance est du potassium.

6. Dispositif de traitement du sang selon l'une des revendications précédentes, **caractérisé en ce que** la deuxième plage de valeurs s'étend de nul à une valeur maximale.

7. Dispositif de traitement du sang selon l'une des revendications précédentes, **caractérisé en ce que** dans l'unité d'évaluation (32), une valeur cible Mend pour la quantité totale à retirer figure dans la troisième plage de valeurs.

8. Dispositif de traitement du sang selon la revendication 7, **caractérisé en ce que** l'unité de d'évaluation (32) est ainsi conçue qu'elle ordonne à l'unité de commande (34) que la valeur cible Mend soit atteinte après un temps de traitement prévu.

9. Dispositif de traitement du sang selon la revendication 7, **caractérisé en ce que** l'unité d'évaluation (32) est ainsi conçue qu'elle ordonne à l'unité de commande (34), lorsque la valeur cible Mend est atteinte, de poursuivre le traitement du sang avec une concentration Cdi de la substance dans la tubulure d'alimentation de liquide de dialyse (22) de façon à ce qu'il n'y ait plus de transfert de la substance à travers la membrane (3).

10. Dispositif de traitement du sang selon l'une des revendications précédentes, **caractérisé en ce que** l'unité de commande (34) est adaptée pour ordonner une mesure initiale de la concentration sanguine Cbi avec des paramètres de traitement préréglés et l'unité de d'évaluation (32) est adaptée pour déterminer la valeur initiale de Cbi et en tenant compte de cette valeur, de la première plage de valeurs autorisée et de la deuxième plage de valeurs autorisée pour le traitement du sang, de proposer une valeur pour la concentration Cdi de la substance dans la tubulure d'alimentation de liquide de dialyse (22), du débit de liquide de dialyse Qd et/ou de la circulation sanguine Qb.

**11.** Dispositif de traitement du sang selon la revendication 10, **caractérisé en ce que** l'unité d'évaluation (32) est ainsi conçue qu'elle détermine la concentration Cdi par la valeur correspondant à la limite inférieure de la première plage de valeurs autorisée.

**12.** Dispositif de traitement du sang selon la revendication 10, **caractérisé en ce que** l'unité d'évaluation (32) est ainsi conçue qu'elle détermine la concentration Cdi par la limite supérieure de la deuxième plage de valeurs autorisée.

**13.** Dispositif de traitement du sang selon la revendication 11 et 12, **caractérisé en ce que** sur un dispositif de saisie (35), des moyens de sélection sont prévus par une prioritisation du retrait par un alignement sur la limite inférieure de la première plage de valeurs autorisée ou sur la limite supérieure de la deuxième plage de valeurs autorisée.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 4244787 A
- US 4508622 A
- EP 0330892 A2
- WO 9823311 A
- US 5100554 A
- DE 10317024
- US 6126831 A
- DE 10212247

### In der Beschreibung aufgeführte Nicht-Patentliteratur

- Clinical Expression of Potassium Disturbances. **J.P. Knochel.** The Regulation of Potassium Balance. 1989, 207-240
- **E.G. Lowrie ; N.L. Lew.** Death Risk in Hemodialysis Patients: The Predictive Value of Commonly Measured Variables and an Evaluation of Death Rate Differences Between Facilities. *Am.J.Kidney Dis.,* 1990, vol. 15, 458-482
- Microprocessor based universal dialysis calculator for individualization of artificial kidney dialysis. **S. Stiller ; H. Mann ; F. Raab.** MEDINFO. North-Holland Publishing Company, 1980, vol. 80, 534-538
- Principles and biophysics. **J.A. Sargent ; F.A. Gotch et al.** Replacment of Renal Function by Dialysis. Kluwer Academic Publishers, 1996, 34ff